# EUROPEAN PATENT SPECIFICATION

(11) **EP 3 560 460 B1**
(45) Date of publication and mention of the grant of the patent: **06.09.2023**
(21) Application number: 17886517.6
(22) Date of filing: 23.10.2017
(51) Int. Cl.: A61F 2/954, A61F 2/07

(54) **INTRAOPERATIVE STENT DELIVERY SYSTEM AND INTRAOPERATIVE STENT SYSTEM**
INTRAOPERATIVES STENTEINFÜHRUNGSSYSTEM UND INTRAOPERATIVES STENTSYSTEM
SYSTÈME DE POSE DE STENT PEROPÉRATOIRE ET SYSTÈME DE STENT PEROPÉRATOIRE

(30) Priority: 26.12.2016 CN 201611218484
(43) Date of publication of application: 30.10.2019
(73) Proprietor: Shanghai MicroPort Endovascular MedTech (Group) Co., Ltd., Shanghai 201318 (CN)
(72) Inventor: WANG, Liwen, Shanghai 201318 (CN); YUAN, Zhenyu, Shanghai 201318 (CN); ZHU, Qing, Shanghai 201318 (CN); PENG, Dadong, Shanghai 201318 (CN); LI, Zhonghua, Shanghai 201318 (CN); MIAO, Zhenghua, Shanghai 201318 (CN)
(74) Representative: Patentship Patentanwaltsgesellschaft mbH
(86) International application number: PCT/CN2017/107327
(87) International publication number: WO 2018/121016

(56) References cited:
- EP-A1- 1 913 897
- EP-A2- 1 920 734
- WO-A1-2016/123676
- WO-A1-2016/123676
- CN-A- 101 897 629
- CN-A- 104 873 305
- CN-A- 106 618 822
- US-A1- 2006 142 835
- US-A1- 2008 228 260
- US-A1- 2009 099 648
- US-A1- 2009 099 648
- US-A1- 2010 121 429
- US-A1- 2016 158 043

## Description

### TECHNICAL FIELD

The present invention pertains to the technical field of medical devices and particularly relates to a surgical stent delivery system and a surgical stent system.

### BACKGROUND

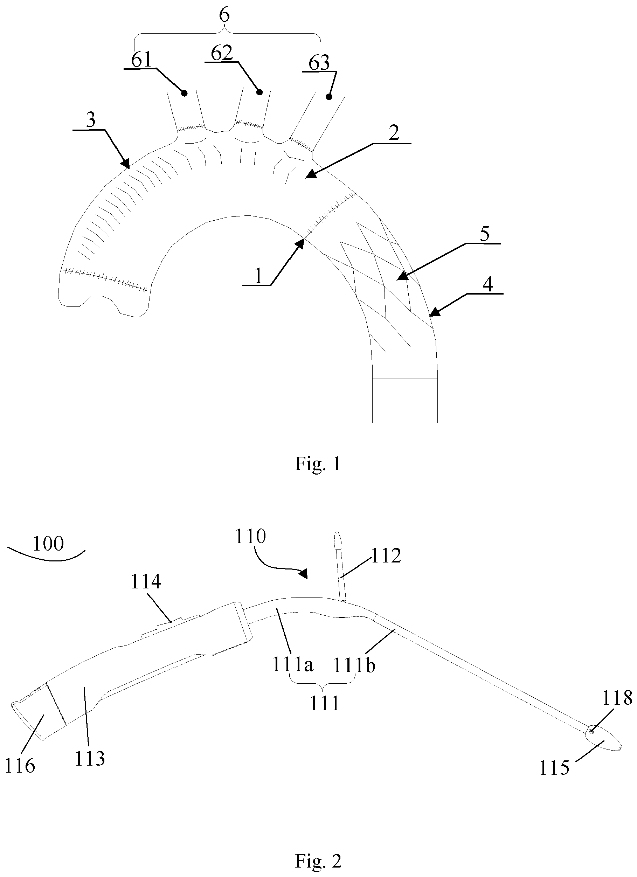

Nowadays, an aortic disease affecting the ascending aorta, the aortic arch and part of the descending aorta is commonly treated by surgical implantation of a stent. The surgical stent is attached, at its proximal end, with a four-branched or straight vascular graft. As shown in Fig. 1, the aortic disease is usually treated by a so-called "elephant trunk" procedure in which the chest is opened and an incision is made in the aortic arch, followed by implanting the stent 5 in the descending aorta 4 and attaching the vascular graft 2 to the proximal end of the stent 5 so as to replace the diseased ascending aorta and aortic arch 3. At last, the vascular graft 2 is anastomosed to the native blood vessels.

However, this traditional "elephant trunk" procedure is challenged by the difficulties in mobilizing and anastomosing the three branches of blood vessels 6 of the aortic arch 3 (i.e., the innominate artery 61, the left common carotid artery 62 and the left subclavian artery 63), especially the left subclavian artery 63 which is located deeper in the chest cavity and thus difficult to access or even visualize. For this reason, anastomosing the vascular graft 2 to the left subclavian artery 63 is a troublesome, time-taking operation with a high risk of causing damage to the recurrent laryngeal and radial nerves.

As an alternative approach to the "elephant trunk" procedure, a trifurcated stent may be used, which does not require anastomosing the three branches 6 of the aortic arch 3 and thus can simplify operations and reduce complications caused by cardiopulmonary bypass (CPB) and deep hypothermic circulatory arrest (DHCA). However, given the fact that there are individual differences in vascular anatomy structures, the three branches may be located at different positions among different patients, making it almost impossible for any such existing trifurcated stent to exactly fit a patient's vascular geometry. As a result, narrowing or even clogging of one or more of the branches or dislodgement of the entire stent may occur over time. Further, since any particular design of such trifurcated stents is not applicable to different patients, they are lack of generality.

US 2009/099648 A1 discloses a modular aortic stent graft and a modular branched stent graft, expandable between a compressed condition and an expanded condition, for assembling with each other at the treatment area of an aneurysm. EP 1 920 734 A2 discloses a modular aortic stent graft and a modular branched stent graft, expandable between a compressed condition and an expanded condition, for assembling with each other at the treatment area of an aneurysm. US 2016/0158043 A1 discloses a system for deploying a stent graft at a branched artery. WO 2016/123676 A1 discloses an aortic stent graft configured for placement in the aortic arch and a delivery system to enable endovascular delivery of the aortic stent graft.

### SUMMARY OF THE INVENTION

The invention is set out in the appended set of claims.

It is an object of the present invention to present a surgical stent delivery system and a surgical stent system so as to solve the problem that conventional surgical stents are difficult to be anastomosed to the aortic arch branches, especially the left subclavian artery, so as to achieve easier thoracotomy operations, shorter overall operation time, fewer postoperative complications and a higher success rate and better postoperative outcome of thoracotomy, as well as a widened scope of suitable patients.

The above and other related objects are attained by a surgical stent delivery system for loading and delivering a surgical stent, which comprises a main liner, a branch liner disposed on the main liner and an operating handle in communication with the main liner, wherein the branch liner is rotatably disposed on the main liner.

Preferably, in the surgical stent delivery system, the main liner may comprise a first portion and a second portion in communication with each other. The branch liner is rotatably arranged on the first portion. The operating handle is in communication with the first portion.

The surgical stent delivery system further comprises a pivoting adjustment member. The pivoting adjustment member is provided on the operating handle and is coupled to the branch liner so as to drive the branch liner to rotate with respect to the main liner.

In the surgical stent delivery system, the pivoting adjustment member comprises a slider assembly and a linkage assembly, wherein the slider assembly comprises a slider slidably disposed on the operating handle and a base connected to the slider, wherein the slider and the base are respectively located outside and inside the operating handle, and wherein a first part of the linkage assembly received within the operating handle and coupled to the base and a second part of the linkage assembly received within the first portion of the main liner and coupled to the branch liner.

In the surgical stent delivery system, the first part of the linkage assembly is rotatably or fixedly coupled to the base and the second part of the linkage assembly is rotatably coupled to the branch liner.

Preferably, in the surgical stent delivery system, an aperture is arranged in a wall of the first portion of the main liner, a rotating shaft is fixedly attached to the wall of the first portion at the aperture, wherein an end of the branch liner is rotatably coupled to the rotating shaft, and wherein the second part of the linkage assembly is rotatably coupled to the end of the branch liner on one side of the rotating shaft so that the branch liner is rotatable about the rotating shaft under driving of the linkage assembly.

Preferably, in the surgical stent delivery system, the rotating shaft may be integral with the first portion of the main liner.

Preferably, in the surgical stent delivery system, the end of the branch liner may be coupled to a connecting shaft in which a first hole and a second hole are arranged with a spacing therebetween, wherein the linkage assembly has a protrusion projecting therefrom, and wherein the rotating shaft is moveably received in the first hole and the protrusion is moveably received in the second hole.

Preferably, in the surgical stent delivery system, the first portion of the main liner may be curved axially, with the second part of the linkage assembly received within the first portion being curved axially.

Preferably, the surgical stent delivery system may further comprise a pre-bent tube configured to be inserted into the second portion of the main liner so as to change a curvature of the second portion by the pre-bent tube.

The above and other related objects are also attained by a surgical stent system comprising a surgical stent and the surgical stent delivery system as defined in any of the above paragraphs. The surgical stent comprises a main stent and a branch stent that is disposed on the main stent and projects from an outer circumference thereof. The branch stent is configured to be sleeved over the branch liner, and the main stent is configured to be sleeved over the main liner.

In the surgical stent system, the surgical stent further comprises a soft cover covering the main stent and/or the branch stent.

In the surgical stent system, the surgical stent further comprises a vascular graft coupled and in communication with a proximal end of the main stent, with the vascular graft configured to be sleeved over the main liner.

In summary, the surgical stent system and the surgical stent delivery system of the present invention offer the following advantages:
1. By arranging the branch liner that is rotatable with respect to the main liner, the branch stent matching the branch liner can rotate in synchronization with respect to the main stent matching the main liner. Therefore, the angle between the branch stent and the main stent can be adjusted during the surgery so as to quickly and accurately match the actual angle between the left subclavian artery and the aortic arch (or the descending aorta). This can facilitate the intraoperative alignment between the branch stent and the left subclavian artery, make the implantation of the surgical stent easier, avoid too much time spent in mobilizing and anastomosing the left subclavian artery during the open-chest procedure, lower operational difficulties, shorten the time required for cardiopulmonary bypass (CPB) and deep hypothermic circulatory arrest (DHCA) and significantly minimize the overall operation cycle time.
2. Thanks to the adjustable angle between the branch stent and the main stent, one type of surgical stent is able to address various anatomy structures of different patient populations, thereby lowering the production cost of the surgical stent, widening its scope of suitable patients, enhancing its therapeutic efficacy, lessening postoperative complications and increasing the success rate of open-chest treatment of aortic diseases.
3. By inserting the pre-bent tube into the main liner, the curvature of the main stent can be altered so that the surgical stent may better conform to the actual three-dimensional vascular anatomy, which can facilitate surgical operations and improve the implantation of the surgical stent.
4. The main stent and/or branch stent is covered by the soft cover that constraining the main stent and/or branch stent has a rather smooth surface, thereby avoiding vascular damage during delivery of the surgical stent.

### BRIEF DESCRIPTION OF THE DRAWINGS

Fig. 1 is a schematic diagram explaining the principles of a conventional "elephant trunk" procedure employing a surgical stent.
Fig. 2 is a structural schematic view of a surgical stent delivery system according to an embodiment of the present invention, showing an external wire puller being coupled to an operating handle.
Fig. 3 is a structural schematic view of a surgical stent according to an embodiment of the present invention.
Fig. 4 schematically illustrates a branch liner of a surgical stent delivery system that has been pivoted counterclockwise by a first angle in accordance with an embodiment of the present invention.
Fig. 5 schematically illustrates a branch liner of a surgical stent delivery system that has been pivoted clockwise by a second angle in accordance with an embodiment of the present invention.
Fig. 6 is a partially cross-sectional view of a branch liner of a surgical stent delivery system that has been pivoted counterclockwise by a certain angle in accordance with an embodiment of the present invention.
Fig. 7 is a partially cross-sectional view of a branch liner of a surgical stent delivery system that has been pivoted clockwise by a certain angle in accordance with an embodiment of the present invention.
Fig. 8 is a structural schematic view of a surgical stent delivery system according to an embodiment of the present invention, showing an external wire puller being detached from an operating handle.
Fig. 9 is a schematic illustration of a surgical stent delivery system according to an embodiment of the present invention, showing a surgical stent being loaded thereon.
Fig. 10 is a schematic illustration of a surgical stent delivery system according to an embodiment of the present invention in a configuration suitable for deployment of a surgical stent.
Fig. 11 schematically illustrates a surgical stent according to an embodiment of the present invention that has been deployed and anchored in the aorta and the left subclavian artery.

### List of Reference Numerals in Drawings

- 100: surgical stent delivery system
- 111: main liner
111a first portion
111b second portion

- 112: branch liner
112a first hole
112b second hole

- 113: operating handle
- 114: pivoting adjustment member
1141 slider assembly
1141a slider
1141b base
1142 linkage assembly

- 115: fixed tip
- 116: external wire puller
- 117: internal wire puller
- 118: guide hole
- 200: surgical stent
210 main stent
220 branch stent
230 soft cover
240 vascular graft
250 three-branched vascular graft

- θ1: first angle
- θ2: second angle
- X, Y, Z: positions of branch liner

### DETAILED DESCRIPTION

The core concept of the present invention is to provide a surgical stent system including a surgical stent and a surgical stent delivery system. The surgical stent includes a main stent and a branch stent. The branch stent is arranged on the main stent and projects from an outer circumference thereof. The surgical stent delivery system is used to load and deliver the surgical stent and includes a main liner, a branch liner arranged on the main liner and an operating handle in communication with the main liner. In particular, the branch liner is rotatably arranged on the main liner; the branch stent is configured to be sleeved over the branch liner; the main stent is configured to be sleeved over the main liner. In this way, when the branch liner rotates with respect to the main liner, the branch stent is driven to rotate in synchronization with respect to the main stent. Thus, the angular orientation of the branch stent can be adjusted as desired, which can facilitate intraoperative alignment between the branch stent and the left subclavian artery, make implantation easier, lower operational difficulties, reduce long-term complications, avoid increased postoperative complications from too much time spent in mobilizing and anastomosing the left subclavian artery and enhance the efficacy of open-chest treatment of aortic diseases.

The objects, advantages and features of the invention will become more apparent upon reading the following detailed description of the proposed surgical stent system and surgical stent delivery system in connection with the Figs. 2 to 11. Note that the figures are much simplified and may not be drawn to scale, and their sole purpose is to facilitate easy and clear explanation of some embodiments of the invention.

As used herein and in the appended claims, the singular forms of "a," "an", and "the", include plural references unless the context clearly dictates otherwise. As used herein and in the appended claims, the term "or" refers to "and/or" unless the context clearly dictates otherwise. In addition, the term "proximal end" generally refers to an end close to the heart, and "distal end" to an end refers to an end away from the heart. Throughout the figures, like reference numerals indicate the same or analogous components or elements.

Reference is now made to Fig. 2, a structural schematic view of a surgical stent delivery system 100 according to an embodiment of the present invention. As shown, the surgical stent delivery system 100 includes a main liner 111, a branch liner 112 and an operating handle 113 and is configured to load and deliver a surgical stent 200. The branch liner 112 is disposed on the main liner 111 and may project laterally outward from an outer circumference thereof. The operating handle 113 may be disposed at a proximal end of the main liner 111 and be in communication therewith. In one embodiment of the present invention, the operating handle 113 may be structured as a housing and manipulated by an operator to control the delivery, loading, positioning and deployment of the surgical stent 200, as well as any other operations related to the implantation of the surgical stent 200.

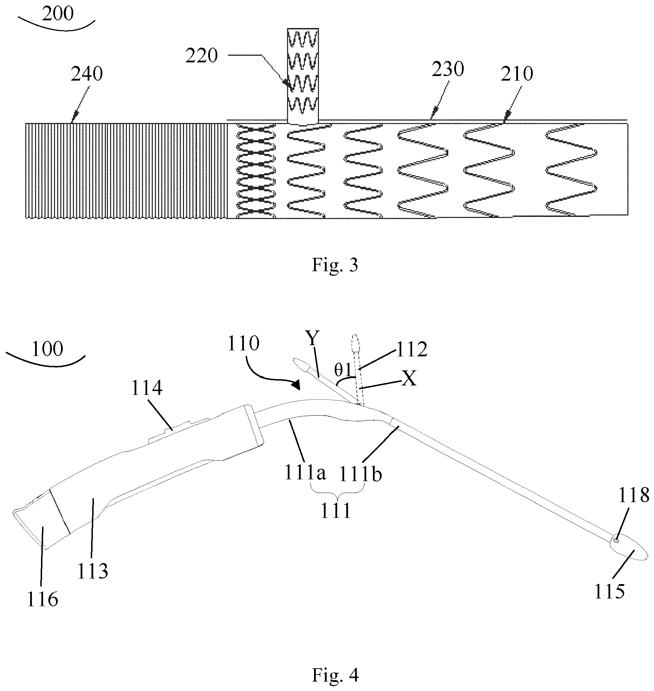

As an implant, the surgical stent 200 is used to treat an aortic disease affecting the ascending aorta, the aortic arch and the descending aorta by creating a blood flow path which alters the blood flow direction in a diseased native vessel. As shown in Fig. 3, the surgical stent 200 of the present invention includes a main stent 210 and a branch stent 220. The branch stent 220 is arranged on the main stent 210 and projects laterally outward from an outer circumference thereof. In a preferred embodiment, the surgical stent 200 further includes a soft cover 230 and a vascular graft 240. As a preferred structure, the soft cover 230 may cover the main stent 210 and/or the branch stent 220. As an optional structure, the vascular graft 240 may be configured to be sleeved over the main liner 111 as well as coupled and in communication with a proximal end of the main stent 210. The vascular graft 240 is preferably a coated vascular graft.

During assembly, the main liner 111 may be inserted into the main stent 210, and the branch liner 112 into the branch stent 220. After that, the surgical stent delivery system 100 is manipulated to deliver the surgical stent 200 into the body of a patient. It is to be noted that, the surgical stent 200 needs to be crimped onto the liners of the surgical stent delivery system 100 by using an external component so as to be delivered into the body. In this way, after the surgical stent 200 is delivered to the target lesion site, it can be deployed by undoing the crimping by the external component. In one embodiment, the external component may be preferably the aforementioned soft cover 230 so as to tighten the corresponding stent.

In particular, the branch liner 112 is pivotably arranged on the main liner 111 so that the branch stent 220 disposed over the branch liner 112 can pivot with respect to the main stent 210 over the main liner 111 in synchronization with the pivoting of the branch liner 112.

In a preferred embodiment, the main liner 111 may include a first portion 111a and a second portion 11 1b. Both of the first portion 111a and the second portion 11 1b are hollow and in communication with each other. The first portion 111a may be curved axially so as to better conform to the anatomy of the aortic arch.

In addition, the second portion 111b may be disposed at a distal end of the main liner 111. The first portion 111a may be disposed at the proximal end of the main liner 111 and be in communication with the operating handle 113. In this case, the branch liner 112 may be pivotably arranged on the first portion 111a. It is to be noted that the first portion 111a is configured to fit the aortic arch so as to simultaneously bring the branch stent 220 of the surgical stent 200 and the second portion 111b into fit with the left subclavian artery and the descending aorta, respectively. In this way, better positioning of the surgical stent 200 in the aorta can be achieved. Optionally, the first and second portions 111a, 111b may either be joined together by riveting or integral with each other, and the present invention is not particularly limited in this regard.

The surgical stent delivery system 100 may further include a pivoting adjustment member 114 provided on the operating handle 113 and be coupled to the branch liner 112 so as to drive the branch liner 112 to pivot with respect to the main liner 111.

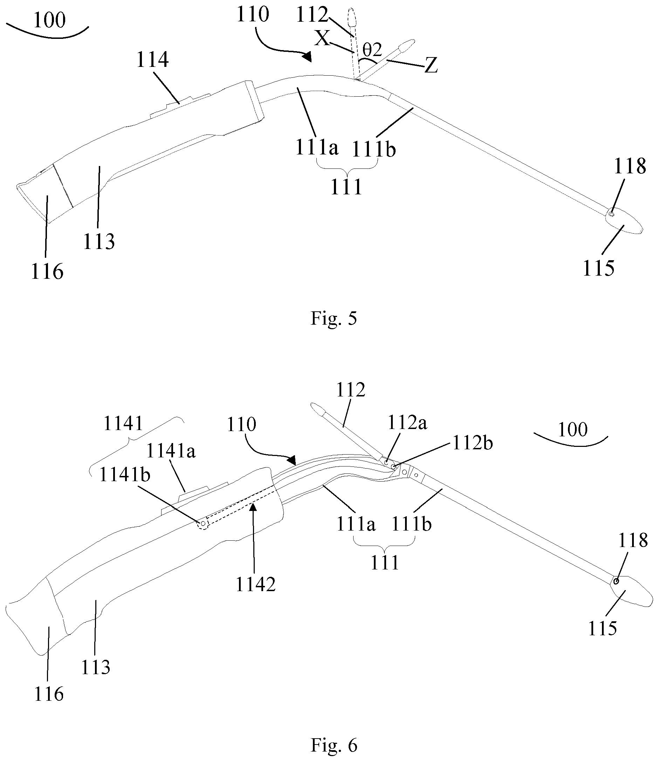

As shown in Figs. 4 and 5, under the driving of the pivoting adjustment member 114, the branch liner 112 is pivotable to the left or right about a pivot axis, resulting in a change in an angle between the branch liner 112 and the main liner 111. Compared to the configuration shown in Fig. 2, the branch liner 112 in Fig. 4 has pivoted counterclockwise (i.e., proximally) by a first angle θ1. Specifically, the branch liner 112 has shifted to the left (proximally) from the position X to the position Y. In Fig. 4, the branch liner 112 prior to the pivoting is shown as a dashed box. Compared to the configuration shown in Fig. 2, the branch liner 112 in Fig. 5 has pivoted clockwise (i.e., distally) by a second angle θ2. Specifically, the branch liner 112 has shifted to the right (distally) from the position X to the position Z. In Fig. 5, the branch liner 112 prior to the pivoting is shown as a dashed box. The present invention is not limited to any particular value of the angle by which the branch liner 112 pivots clockwise or counterclockwise (proximally or distally), and it may be determined according to the actual applications.

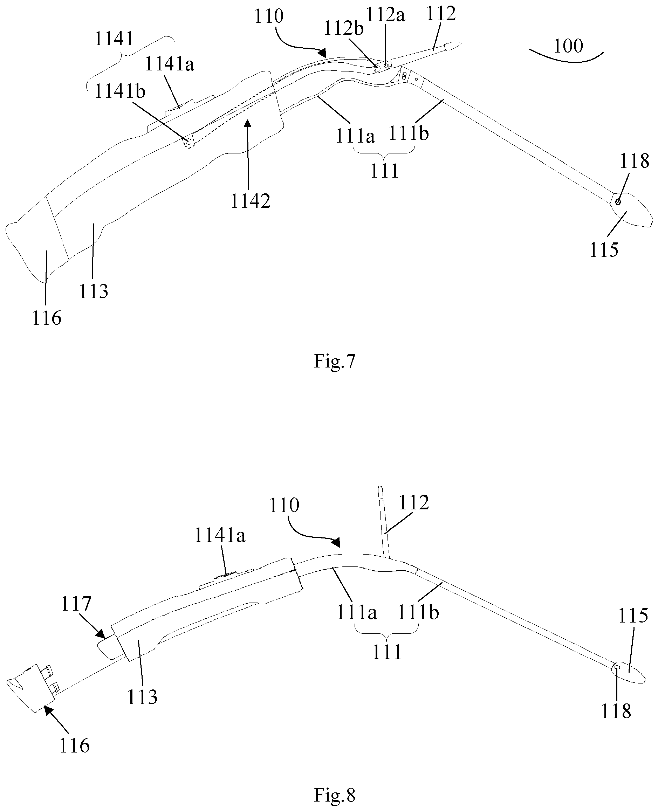

In one embodiment of the present invention, the pivot axis may be defined by a center axis of a rotating shaft. As shown in Figs. 6 and 7, an aperture is arranged on the wall of the first portion 111a. The rotating shaft may be fixedly arranged on the wall of the first portion 111a around an aperture. When one end (i.e., a trailing end) of the branch liner 112 is inserted into the first portion 111a through the aperture, it is engaged with the rotating shaft and the branch liner 112 can pivot about the rotating shaft.

In some embodiments of the present invention, the pivoting adjustment member 114 may include a slider assembly 1141 and a linkage assembly 1142. The slider assembly 1141 may include a slider 1141a and a base 1141b, which are connected to each other and disposed outside and inside the operating handle 113, respectively. The linkage assembly 1142 may consist of a first part received within the operating handle 113 and pivotably or fixedly coupled to the base 1141b and a second part received within the first portion 111a and pivotably coupled to the branch liner 112. Optionally, the operating handle 113 is provided with a sliding slot and the slider 114a may be slidably arranged in the sliding slot.

In one embodiment of the present invention, the branch liner 112 may be pivotably coupled to the rotating shaft, with the linkage assembly 1142 pivotably coupled to the branch liner 112 on one side of the rotating shaft (e.g., on the right side as shown in Fig. 6), so that when the linkage assembly 1142 moves under the driving of the slider assembly 1141, it causes the branch liner 112 to synchronously pivot about the rotating shaft.

In a preferred embodiment, a first hole 112a and a second hole 112b may be arranged in the branch liner 112 with a spacing therebetween. In this case, the branch liner 112 is inserted over the rotating shaft with the aid of the first hole 112a so as to be pivotable about the rotating shaft and coupled to the linkage assembly 1142 with the aid of the second hole 112b so as to be pivotable with respect to the linkage assembly 1142.

In one embodiment of the present invention, the rotating shaft may be integral with the first portion 111a. That is, a circular protrusion may project from the wall of the first portion 111a to act as the rotating shaft. Preferably, the second part of the linkage assembly 1142 received within the first portion 111a may have a curved shape along the axis so as to match the curved first portion 111a such that the linkage assembly is able to smoothly move in the first portion 111a.

In another preferred embodiment, the branch liner 112 may be fixedly coupled to a connecting shaft. The first and second holes 112a, 112b that are spaced apart from each other are arranged on the connecting shaft. The other portion of the linkage assembly 1142 may define a protrusion projecting therefrom. In this case, the rotating shaft integral with the first portion 111a and the protrusion of the linkage assembly 1142 may be inserted into the first and second holes 112a, 112b, respectively, so that the branch liner 112 can pivot under the driving of the linkage assembly 1142.

In one embodiment of the present invention, a third hole may be arranged in the base 1141b. A fourth hole may be arranged in the second part of the linkage assembly 1142 received within the first portion 111a. A pin may be inserted through both the third and fourth holes to pivotably couple the first part of the linkage assembly 1142 to the base 1141b.

With combined reference to Figs. 6 and 7, during practical use of the surgical stent delivery system 100, the slider 1141a may be pushed to proximal or distal ends of the surgical stent delivery system 100 so as to drive the linkage assembly 1142 to move in the same direction and hence the trailing end of the branch liner 112 to move in the same direct. Since the trailing end of the branch liner 112 is received within the main liner 111, with the other end (i.e., the leading end) projecting out of the main liner 111, the leading end of the branch liner 112 will pivot toward the operating handle 113 (i.e., proximally) with the movement of the trailing end of the branch liner 112 toward a fixed tip 115 of the surgical stent delivery system 100 (i.e., distally). For example, when the slider 114a is pushed to the right side of Fig. 6 (i.e., toward the fixed tip 115 of the delivery system), the branch liner 112 will pivot toward the left side (i.e., toward the operating handle 113 of the system); and when the slider 1141a is pushed to the left side of Fig. 7 (i.e., toward the operating handle 113), the branch liner 112 will pivot toward the fixed tip 115 (i.e., to the right side).

In a preferred embodiment, the pivoting adjustment member 114 may further include a limit structure (not shown). When the branch liner 112 pivots to left or right until against the limit structure, the branch liner 112 stops pivoting; or when the linkage assembly 1142 moves toward the fixed tip 115 or the operating handle 113 until against the limit structure, the linkage assembly 1142 stops moving.

Referring to Fig. 8, in addition to the operating handle 113, the stent delivery system 100 may further include an external wire puller 116 and an internal wire puller 117 that are disposed at the proximal end of the operating handle 113. Each of the external wire puller 116 and the internal wire puller 117 is detachable to the operating handle 113. When the external wire puller 116 is coupled to the operating handle 113, a part of the internal wire puller 117 may be received in the external wire puller 116 and the other part may be received in the operating handle 113. Optionally, the external wire puller 116 may be detachably coupled to the operating handle 113 by a snap-on or threaded connection.

The external wire puller 116 is configured to control the deployment of the main stent 210. Specifically, when the external wire puller 116 is pulled to the left of the surgical stent delivery system 100, for example, a binding thread to constrain arranged on the main stent 210 may be loosened under the effect of the external wire puller 116, thereby deploying the main stent 210 to a target site. Similarly, the internal wire puller 117 is configured to control the deployment of the branch stent 220. Likewise, when the external wire puller 117 is pulled to the left of the surgical stent delivery system 100, for example, another binding thread to constrain arranged on the branch stent 220 may be loosened under the effect of the internal wire puller 117, thus deploying the branch stent 220 to a target site.

With continued reference to Fig. 8, the fixed tip 115 may be disposed at a distal end of the second portion 111b. The fixed tip 115 is preferably selected as a tapered structure so that it can not only avoid cause damage to any vascular tissue but also facilitate the delivery due to streamlined surface of the taper. Additionally, the fixed tip 115 may define a guide hole 118. A guide wire may be introduced into a true lumen of the diseased aorta at one end and into the guide hole 118 at the other end. The entire delivery system may be then advanced along the guide wire into the true lumen of the diseased aorta, followed by deployment of the surgical stent 200 therein.

In the foregoing embodiments, preferably, each of the operating handle 113, the external wire puller 116, the internal wire puller 117, the fixed tip 115, the slider 1141a, the branch liner 112 and the main liner 111 may be made of a biocompatible material, such as a polymer material. Specifically, each of the operating handle 113, the external wire puller 116, the internal wire puller 117 and the fixed tip 115 may be made of one or more of PC (polycarbonate) and ABS (acrylonitrile-butadiene-styrene). Each of the slider 1141a, the branch liner 112 and the main liner 111 may be made of a material such as silicone or rubber.

In a preferred embodiment, the surgical stent delivery system 100 may further include a pre-bent tube inserted into the main liner 111. The pre-bent tube may have better deformation performance than the main liner 111 and can help bend the main liner 111 to a curvature required by the target aorta in advance prior to the delivery and implantation of the surgical stent 200. In particular, the pre-bent tube may be configured to be inserted into the second portion 111b of the main liner 111 so as to bend the second portion 111b to a desired curvature for the target descending aorta in advance, thus changing the overall curvature of the main liner 111. This is helpful in adapting the surgical stent 200 to the actual three-dimensional vascular anatomy and improving its deployment effect.

In one embodiment, the pre-bent tube may have an outer diameter ranging from 1.0 mm to 1.8 mm, more preferably 1.0 mm, 1.2 mm, 1.5 mm or 1.8 mm. Here, the diameter of the pre-bent tube is so chosen based on the present inventors' consideration that a greater diameter will increase the effort required to create the curvature of the pre-bent tube, while a smaller diameter will make the pre-bent tube less rigid and easily deformable, which is unfavorable to the delivery. The pre-bent tube is preferably made of stainless steel.

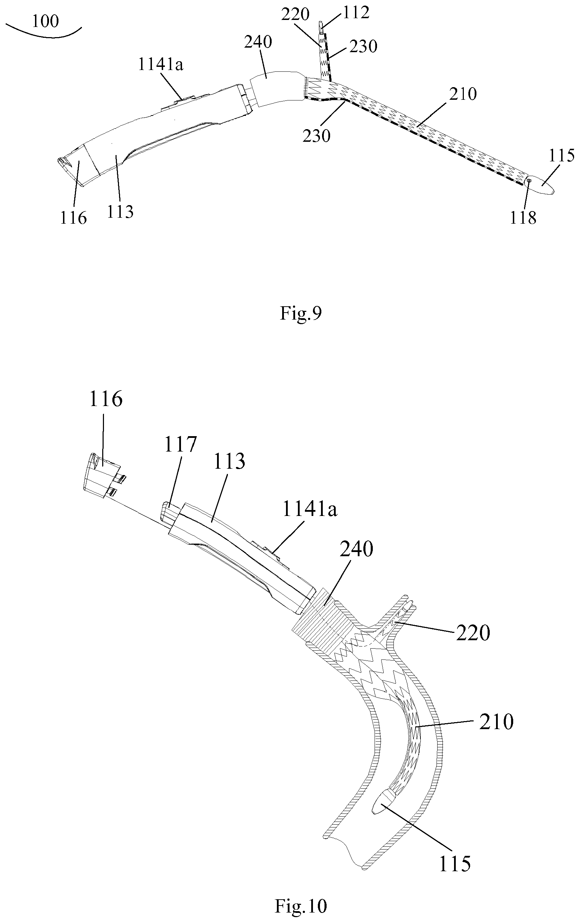

In one embodiment of the present invention, the soft cover 230 may be configured to collaborate with the binding threads to constrain the surgical stent 200 so that the surgical stent 200 can be loaded in the surgical stent delivery system 100 and delivered into the patient's body, as shown in Fig. 9. Concerning the soft cover 230, it may have another function. For example, during implantation of the surgical stent 200 wrapped by the soft cover 230, the surface smoothness of the surgical stent 200 may be increased, so that vascular damage may be avoided and the advancement and delivery of the surgical stent 200 may be facilitated.

A process to deliver the surgical stent 200 will be described below with combined reference to Figs. 9 to 11.

At first, a preoperative assessment may be performed to determine the curvatures of the aortic arch and the descending aorta. The second portion 111b may be then pre-bent based on the curvature of the descending aorta so that a reasonable matching curvature may be achieved.

Subsequently, observing the position of the left subclavian artery through an incision made in the aortic arch (the incision may be individualized to the patient and usually made longitudinally with respect to the aorta in practice, without being necessarily in the aortic arch), the slider 1141a on the operating handle 113 may be moved forth or back to tune the angle between the branch stent 220 and the main stent 210.

Afterward, the guide wire may be inserted into the true lumen of the diseased aorta at one end and the guide hole 118 of the fixed tip 115 at the other end. The surgical stent delivery system 100 loaded with the surgical stent 200 may be then delivered along the guide wire to the lesion site.

After arrival at the lesion site, the branch stent 220 may be positioned in the left subclavian artery, and the main stent 210 and the branch stent 220 may be then sequentially deployed by manipulating the external wire puller 116 and the internal wire puller 117, respectively, as shown in Fig. 10.

Finally, the surgical stent delivery system 100 may be retrieved subsequent to the deployment of the surgical stent 200.

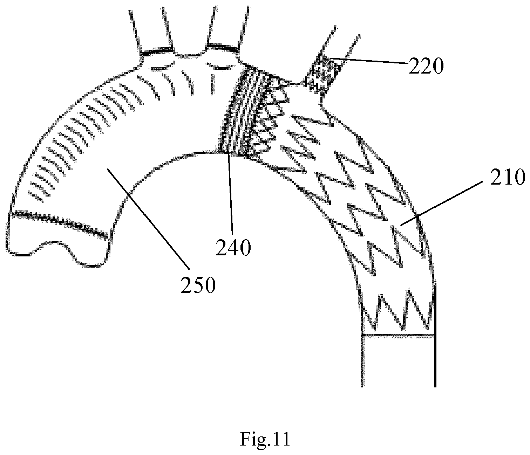

In case of the surgical stent 200 attached with the vascular graft 240, subsequent to the retrieval of the surgical stent delivery system 100, it is necessary to suture the vascular graft 240 to a three-branched vascular graft 250 and then anastomose the three-branched vascular graft to the native vessels. As a result, as shown in Fig. 11, a new blood flow path will be created and the diseased aneurysmal lumen will be isolated from the blood circulation system so that the diseased aneurysmal wall will no longer be subject to blood-pressure pulsations, thereby preventing any rupture of the aneurysm and achieving the therapeutic goals.

In one embodiment, the main stent 210 may include a body of the main stent and a main graft covering the main body of the main stent. The branch stent 220 may include a body of the branch stent and a lateral graft covering the body of the branch stent. In this case, the soft cover 230 may cover the main graft or lateral graft.

It is to be noted that, despite the preferred embodiments disclosed hereinabove, the present invention is not limited to the scope of these disclosed embodiments. For example, while the pivoting adjustment member has been described above as driving the branch liner to pivot by virtue of the combination of the slider assembly and the linkage assembly, the present invention is not so limited because any other approach capable of causing the pivoting of the branch liner is also possible, as long as it allows suitable loading and delivery of the surgical stent.

Compared with the prior art, the present invention offers the following advantages:
1. By arranging the branch liner that is rotatable with respect to the main liner, the branch stent matching the branch liner can rotate in synchronization with respect to the main stent matching the main liner. Therefore, the angle between the branch stent and the main stent can be adjusted during the surgery so as to quickly and accurately match the actual angle between the left subclavian artery and the aortic arch (or the descending aorta). This can facilitate the intraoperative alignment between the branch stent and the left subclavian artery, make the implantation of the surgical stent easier, avoid too much time spent in mobilizing and anastomosing the left subclavian artery during the open-chest procedure, lower operational difficulties, shorten the time required for cardiopulmonary bypass (CPB) and deep hypothermic circulatory arrest (DHCA) and significantly minimize the overall operation cycle time.
2. Thanks to the adjustable angle between the branch stent and the main stent, one type of surgical stent is able to address various anatomy structures of different patient populations, thereby lowering the production cost of the surgical stent, widening its scope of suitable patients, enhancing its therapeutic efficacy, lessening postoperative complications and raising the success rate of open-chest treatment of aortic diseases.
3. By inserting the pre-bent tube into the main liner, the curvature of the main stent can be altered so that the surgical stent may better conform to the actual three-dimensional vascular anatomy, which can facilitate surgical operations and improve the implantation of the surgical stent.
4. The main stent and/or branch stent is covered by the soft cover that constraining the main stent and/or branch stent. As the soft cover has a rather smooth surface, it improves the smoothness of the surface of the stent, c avoiding vascular damage during delivery of the surgical stent.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. A surgical stent system comprising a surgical stent (200) and a surgical stent delivery system (100) for loading and delivering the surgical stent (200), the surgical stent delivery system (100) comprising a main liner (111), a branch liner (112) disposed on the main liner (111), an operating handle (113) in communication with the main liner (111) and a pivoting adjustment member (114) that is provided on the operating handle (113) and coupled to the branch liner (112) so as to drive the branch liner (112) to rotate with respect to the main liner (111), wherein the branch liner (112) is rotatably disposed on the main liner (111);
wherein the pivoting adjustment member (114) comprises a slider assembly (1141) and a linkage assembly (1142), the slider assembly (1141) comprising a slider (1141a) slidably disposed on the operating handle (113) and a base (1141b) connected to the slider (1141a), the slider (1141a) and the base (1141b) respectively located outside and inside the operating handle (113);
wherein a first part of the linkage assembly (1142) is rotatably or fixedly coupled to the base (1141b) and a second part of the linkage assembly (1142) is rotatably coupled to the branch liner (112);
wherein the surgical stent (200) comprising a main stent (210) and a branch stent (220) that is disposed on the main stent (210) and projects from an outer circumference of the main stent (210), the branch stent (220) configured to be sleeved over the branch liner (112), the main stent (210) configured to be sleeved over the main liner (111);
wherein the surgical stent (200) further comprises a soft cover (230) covering the main stent (210) and/or the branch stent (220);
wherein the surgical stent (200) further comprises a vascular graft (240) coupled and in communication with a proximal end of the main stent (210), the vascular graft (240) configured to be sleeved over the main liner (111).

2. The surgical stent system of claim 1, wherein the main liner (111) comprises a first portion (111a) and a second portion (111b) in communication with each other, the branch liner (112) rotatably disposed on the first portion (111a), the operating handle (113) being in communication with the first portion (111a).

3. The surgical stent system of claim 2, wherein the first part of the linkage assembly (1142) is received within the operating handle (113), and the second part of the linkage assembly (1142) is received within the first portion (111a) of the main liner (111).

4. The surgical stent system of claim 3, wherein an aperture is arranged in a wall of the first portion (111a) of the main liner (111), a rotating shaft is fixedly attached to the wall of the first portion (111a) at the aperture, wherein an end of the branch liner (112) is rotatably coupled to the rotating shaft, and wherein the second part of the linkage assembly (1142) is rotatably coupled to the end of the branch liner (112) on one side of the rotating shaft so that the branch liner (112) is rotatable about the rotating shaft under driving of the linkage assembly (1142).

5. The surgical stent system of claim 4, wherein the rotating shaft is integral with the first portion (111a) of the main liner (111).

6. The surgical stent system of claim 4, wherein the end of the branch liner (112) is coupled to a connecting shaft in which a first hole (112a) and a second hole (112b) are arranged with a spacing therebetween; the linkage assembly (1142) has a protrusion projecting therefrom; and the rotating shaft is moveably received in the first hole (112a) and the protrusion is moveably received in the second hole (112b).

7. The surgical stent system of claim 3, wherein the first portion (111a) of the main liner (111) is curved axially, and the second part of the linkage assembly (1142) received within the first portion (111a) is curved axially.

8. The surgical stent system of claim 2, further comprising a pre-bent tube configured to be inserted into the second portion (111b) of the main liner (111) so as to change a curvature of the second portion (111b) by the pre-bent tube.

## Patentansprüche

1. Chirurgisches Stentsystem, umfassend einen chirurgischen Stent (200) und ein chirurgisches Stenteinführungssystem (100) zum Laden und Zuführen des chirurgischen Stents (200), wobei das chirurgische Stenteinführungssystem (100) einen Hauptliner (111), einen an dem Hauptliner (111) angeordneten Zweigliner (112), einen mit dem Hauptliner (111) in Verbindung stehenden Betätigungsgriff (113) und ein schwenkbares Einstellelement (114) umfasst, das auf dem Betätigungsgriff (113) vorgesehen und mit dem Zweigliner (112) gekoppelt ist, um den Zweigliner (112) in Bezug auf den Hauptliner (111) zum Drehen anzutreiben, wobei der Zweigliner (112) drehbar auf dem Hauptliner (111) angeordnet ist;
wobei das schwenkbare Einstellelement (114) eine Schieberbaugruppe (1141) und eine Verbindungsbaugruppe (1142) umfasst, wobei die Schieberbaugruppe (1141) einen verschiebbar auf dem Betätigungsgriff (113) angeordneten Schieber (1141a) und eine mit dem Schieber (1141a) verbundene Basis (1141b) umfasst, wobei der Schieber (1141a) und die Basis (1141b) entsprechend außerhalb und innerhalb des Betätigungsgriffs (113) angeordnet sind;
wobei ein erster Teil der Verbindungsbaugruppe (1142) drehbar oder fest mit der Basis (1141b) gekoppelt ist und ein zweiter Teil der Verbindungsbaugruppe (1142) drehbar mit dem Zweigliner (112) gekoppelt ist;
wobei der chirurgische Stent (200) einen Hauptstent (210) und einen Zweigstent (220) umfasst, der auf dem Hauptstent (210) angeordnet ist und von einem Außenumfang des Hauptstents (210) vorsteht, wobei der Zweigstent (220) ausgebildet ist, über den Zweigliner (112) gehülst zu werden, wobei der Hauptstent (210) ausgebildet ist, über den Hauptliner (111) gehülst zu werden;
wobei der chirurgische Stent (200) ferner ein Softcover (230) umfasst, das den Hauptstent (210) und/oder den Zweigstent (220) bedeckt;
wobei der chirurgische Stent (200) ferner ein mit einem proximalen Ende des Hauptstents (210) gekoppeltes und in Verbindung stehendes Gefäßtransplantat (240) umfasst, wobei das Gefäßtransplantat (240) ausgebildet ist, über den Hauptliner (111) gehülst zu werden.

2. Chirurgisches Stentsystem nach Anspruch 1, wobei der Hauptliner (111) einen ersten Abschnitt (111a) und einen zweiten Abschnitt (111b) umfasst, die miteinander in Verbindung stehen, wobei der Zweigliner (112) drehbar auf dem ersten Abschnitt (111a) angeordnet ist, wobei der Betätigungsgriff (113) mit dem ersten Abschnitt (111a) in Verbindung steht.

3. Chirurgisches Stentsystem nach Anspruch 2, wobei der erste Teil der Verbindungsbaugruppe (1142) in dem Betätigungsgriff (113) aufgenommen ist, und wobei der zweite Teil der Verbindungsbaugruppe (1142) in dem ersten Abschnitt (111a) des Hauptliners (111) aufgenommen ist.

4. Chirurgisches Stentsystem nach Anspruch 3, wobei eine Öffnung in einer Wand des ersten Abschnitts (111a) des Hauptliners (111) angeordnet ist, wobei eine Drehwelle fest an der Wand des ersten Abschnitts (111a) an der Öffnung angebracht ist, wobei ein Ende der Zweigliner (112) drehbar mit der Drehwelle verbunden ist, und wobei der zweite Teil der Verbindungsbaugruppe (1142) drehbar mit dem Ende der Zweigliner (112) auf einer Seite der Drehwelle verbunden ist, so dass der Zweigliner (112) unter Ansteuerung der Verbindungsbaugruppe (1142) um die Drehwelle drehbar ist.

5. Chirurgisches Stentsystem nach Anspruch 4, wobei die Drehwelle einstückig mit dem ersten Abschnitt (111a) des Hauptliners (111) ausgebildet ist.

6. Chirurgische Stentsystem nach Anspruch 4, wobei das Ende des Zweigliners (112) mit einer Verbindungswelle verbunden ist, in der ein erstes Loch (112a) und ein zweites Loch (112b), mit einem Abstand dazwischen, angeordnet sind; wobei die Verbindungsbaugruppe (1142) einen davon hervorstehenden Vorsprung aufweist; und wobei die Drehwelle beweglich in dem ersten Loch (112a) aufgenommen und der Vorsprung beweglich in dem zweiten Loch (112b) aufgenommen ist.

7. Chirurgisches Stentsystem nach Anspruch 3, wobei der erste Abschnitt (111a) des Hauptliners (111) axial gekrümmt ist und der in dem ersten Abschnitt (111a) aufgenommene zweite Teil der Verbindungsbaugruppe (1142) axial gekrümmt ist.

8. Chirurgisches Stentsystem nach Anspruch 2, ferner umfassend ein vorgebogenes Rohr, das ausgebildet ist, in den zweiten Abschnitt (111b) des Hauptliners (111) eingeführt zu werden, um eine Krümmung des zweiten Abschnitts (111b) durch das vorgebogene Rohr zu verändern.

## Revendications

1. Système de stent chirurgical comprenant un stent chirurgical (200) et le système de mise en place de stent chirurgical (100) pour charger et mettre en place le stent chirurgical (200), le système de mise en place de stent chirurgical (100) comprenant une doublure principale (111), un gaine de dérivation (112) disposée sur la gaine principale (111), une poignée de commande (113) en communication avec la gaine principale (111) et un élément de réglage pivotant (114) qui est prévu sur la poignée de commande (113) et couplé à la chemise de ramification (112) de manière à entraîner la chemise de ramification (112) en rotation par rapport à la chemise principale (111), dans laquelle la chemise de ramification (112) est disposée de manière rotative sur la chemise principale (111);
dans lequel l'élément de réglage pivotant (114) comprend un ensemble curseur (1141) et un ensemble de liaison (1142), l'ensemble curseur (1141) comprenant un curseur (1141a) disposé de manière coulissante sur la poignée de commande (113) et une base (1141b) relié au coulisseau (1141a), le coulisseau (1141a) et la base (1141b) situés respectivement à l'extérieur et à l'intérieur de la poignée de manoeuvre (113);
dans lequel une première partie de l'ensemble de liaison (1142) est couplée de manière rotative ou fixe à la base (1141b) et une deuxième partie de l'ensemble de liaison (1142) est couplée de manière rotative au chemisage de ramification (112);
dans lequel le stent chirurgical (200) comprenant un stent principal (210) et un stent ramifié (220) qui est disposé sur le stent principal (210) et fait saillie à partir d'une circonférence extérieure du stent principal (210), le stent ramifié (220) configuré pour être manchonné sur le revêtement de ramification (112), le stent principal (210) étant configuré pour être manchonné sur le revêtement principal (111);
dans lequel le stent chirurgical (200) comprend en outre une couverture souple (230) recouvrant le stent principal (210) et/ou le stent ramifié (220);
dans lequel le stent chirurgical (200) comprend en outre une greffe vasculaire (240) couplée et en communication avec une extrémité proximale du stent principal (210), la greffe vasculaire (240) étant configurée pour être gainée sur le revêtement principal (111).

2. Système de stent chirurgical selon la revendication 1, dans lequel le revêtement principal (111) comprend une première portion (111a) et une seconde portion (111b) en communication l'une avec l'autre, le revêtement de ramification (112) étant disposé de manière rotative sur la première portion (111a) 111a), la poignée de commande (113) étant en communication avec la première partie (111a).

3. Système de stent chirurgical selon la revendication 2, dans lequel la première partie de l'ensemble de liaison (1142) est reçue à l'intérieur de la poignée de fonctionnement (113), et la deuxième partie de l'ensemble de liaison (1142) est reçue à l'intérieur de la première partie (111a) de la chemise principale (111).

4. Système de stent chirurgical selon la revendication 3, dans lequel une ouverture est agencée dans une paroi de la première partie (111a) du revêtement principal (111), un arbre rotatif est fixé de manière fixe à la paroi de la première partie (111a) au niveau l'ouverture, dans laquelle une extrémité de la chemise de ramification (112) est couplée de manière rotative à l'arbre rotatif, et dans laquelle la deuxième partie de l'ensemble de liaison (1142) est couplée de manière rotative à l'extrémité de la chemise de ramification (112) sur un côté de l'arbre rotatif de sorte que la chemise de ramification (112) peut tourner autour de l'arbre rotatif sous l'entraînement de l'ensemble de liaison (1142).

5. Système de stent chirurgical selon la revendication 4, dans lequel l'arbre rotatif fait partie intégrante de la première partie (111a) du revêtement principal (111).

6. Système de stent chirurgical selon la revendication 4, dans lequel le l'extrémité de la chemise de ramification (112) est couplée à un arbre de liaison dans lequel un premier trou (112a) et un deuxième trou (112b) sont agencés avec un espacement entre eux; l'ensemble de tringlerie (1142) a une saillie faisant saillie à partir de celui-ci; et l'arbre rotatif est reçu de manière mobile dans le premier trou (112a) et la saillie est reçue de manière mobile dans le second trou (112b).

7. Système d'endoprothèse chirurgicale selon la revendication 3, dans lequel la première partie (111a) de la partie principale la chemise (111) est incurvée axialement, et la seconde partie de l'ensemble de liaison (1142) reçue dans la première partie (111a) est incurvée axialement.

8. Système de stent chirurgical selon la revendication 2, comprenant en outre un tube pré-courbé configuré pour être inséré dans la deuxième partie (111b) du tube principal. doublure (111) de manière à modifier une courbure de la deuxième partie (111b) par le tube pré-courbé.
